(19) Europäisches Patentamt
European Patent Office
Office européen des brevets

(11) **EP 1 416 303 B1**

(12) **EUROPEAN PATENT SPECIFICATION**

(45) Date of publication and mention
of the grant of the patent:
**11.08.2010 Bulletin 2010/32**

(21) Application number: **03025037.7**

(22) Date of filing: **30.10.2003**

(51) Int Cl.:
*G02B 6/122* (2006.01)      *G02B 6/124* (2006.01)
*G02B 6/125* (2006.01)      *G02B 6/13* (2006.01)
*G02B 6/138* (2006.01)      *B81B 1/00* (2006.01)
*B81B 7/00* (2006.01)      *G02B 1/11* (2006.01)
*C12Q 1/00* (2006.01)      *B01J 19/00* (2006.01)
*G01N 33/53* (2006.01)      *G01N 27/00* (2006.01)
*C12N 5/00* (2006.01)      *A61L 27/00* (2006.01)
*A45B 25/00* (2006.01)

(54) **Method for manufacturing functional substrates comprising columnar micro-pillars**

Verfahren zur Herstellung funktioneller Substrate, die kolumnare Mikrosäulen aufweisen

Procédé de fabrication de substrats fonctionnels comprenant des micro-piliers colonnaires

(84) Designated Contracting States:
**DE GB**

(30) Priority: **30.10.2002 JP 2002315214**
**30.10.2002 JP 2002315217**

(43) Date of publication of application:
**06.05.2004 Bulletin 2004/19**

(60) Divisional application:
**10006428.6**

(73) Proprietor: **Hitachi, Ltd.**
**Chiyoda-ku**
**Tokyo 100-8280 (JP)**

(72) Inventors:
• **Miyauchi, Akihiro, c/o Hitachi, Ltd.**
**Chiyoda-ku,**
**Tokyo 100-8220 (JP)**
• **Kuwabara, Kosuke, c/o Hitachi, Ltd.**
**Chiyoda-ku,**
**Tokyo 100-8220 (JP)**
• **Ogino, Masahiko, c/o Hitachi, Ltd.**
**Chiyoda-ku,**
**Tokyo 100-8220 (JP)**
• **Yoshida, Hiroshi, c/o Hitachi, Ltd.**
**Chiyoda-ku,**
**Tokyo 100-8220 (JP)**
• **Ando, Takashi, c/o Hitachi, Ltd.**
**Chiyoda-ku,**
**Tokyo 100-8220 (JP)**

(74) Representative: **Beetz & Partner**
**Patentanwälte**
**Steinsdorfstrasse 10**
**80538 München (DE)**

(56) References cited:
**WO-A-01/62887      WO-A-02/43937**
**US-B1- 6 368 871**

• **HASE MASASHI ET AL: "Development of novel method to create two-dimensional photonic crystals" SMART STRUCTURES AND MATERIALS 2000: SMART ELECTRONICS AND MEMS, NEWPORT BEACH, CA, USA, 6-8 MARCH 2000, vol. 3990, 2000, pages 314-325, XP002283665 PROCEEDINGS OF THE SPIE - THE INTERNATIONAL SOCIETY FOR OPTICAL ENGINEERING ISSN: 0277-786X**
• **RAGUIN D H ET AL: "STRUCTURED SURFACES MIMIC COATING PERFORMANCE" LASER FOCUS WORLD, PENNWELL PUBLISHING, TULSA, US, vol. 33, no. 4, April 1997 (1997-04), pages 113-117, XP001092855 ISSN: 1043-8092**

**Description**

FIELD OF THE INVENTION:

The present invention relates to a method for manufacturing a functional element equipped with a group of micro-pillars of organic polymer which can be used for micro-biochips or optical devices

BACKGROUND OF THE INVENTION:

Applied Physics, Vol. 71 (10), pp.1251-1255, 2002 document 1 discloses a technique of forming groups of nano-silicon pillars using clusters of metals such as iron, gold and silver as the self-forming nucleus of a plasma etching mask, and creating a photonic crystal (optical device) by removing a specific cylindrical row. The nano-silicon pillar formed on the silicon substrate have a diameter of 50 nm and a height of 1 $\mu$m, with a cluster cycle (pitch) of 500 nm.

Light having a wavelength of about twice the cycle of the nano-pillars cannot pass between the pillars due to a photonic band gap, but the portion with one row of pillars removed (linear defect) allows light to pass through. Thus, the incoming light travels through the aforementioned linear defect. Combination of optical signals (ADD), decomposition (DROP) or change of traveling direction in a very narrow space is permitted by proper layout of this linear defect. The use of a grating method is preferred when the signal is subjected to DROP. Accordingly, formation of the aforementioned linear defect provides an extremely small optical wave-guide.

Journal Vacuum Science and Technology B Vol. 17 (6), pp. 3197 - 3202, 1999 (document 2) discloses a technique of forming resin-made micro-pillars. The surface of a silicon substrate is coated with a film of a PMMA (polymethyl methacrylate) having a molecular weight of 2000 and a height of 95 nm. A mask of a silicon substrate is placed on the PMMA film through a spacer, where the distance between the PMMA film and the mask is 0.3 $\mu$m. Then the sample is heated at a temperature of 130°C for 5 to 80 min. When it has been heated, micro-pillars are formed on the PMMA film. The mask and the spacer are removed from the sample in the final phase. The diameter of the micro-pillars is several micrometers, and the height is described to be 0.28 to 0.43 $\mu$m although the spacer determines this.

Hase et al (Smart Structures and Materials 2000: Smart Electronic and MEMS, Proceedings of the SPIE Vol. 3990 (2000), pages 314-325) disclose a method of manufacturing a photonic crystal comprising columnar micro-pillars of organic polymer. Uncured polymer is poured into a mold comprising micro-holes. After curing of the polymer, the mold is dissolved in acid.

DESCRIPTION OF THE INVENTION:

The technique disclosed in the aforementioned -document 1 uses an etching method to form a group of micro-pillars of the order of nanometers. The material of the nano-pillars must be removed as volatile gas through reaction with etching gas, and is restricted to such inorganic materials as metals, silicon oxide ($SiO_2$ and silicon nitride ($Si_3N_4$). This method is restricted to the use of dry etching methods including ECR plasma etching.

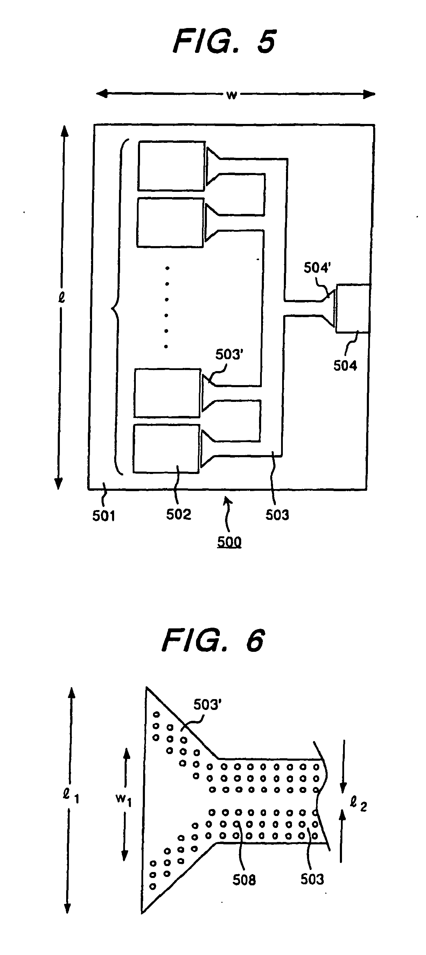

According to the above-mentioned document 1, the diameter of the obtained nano-pillars is 15 to 20 nm, independently of the cluster size (see Fig. 5 of aforementioned document 1). This means that the wavelength of the light used in the optical device obtained according to this method is restricted to a particular range.

Of the techniques described above, the one given in document 2 does not use a dry etching method to form the PMMA micro-pillars. This allows the micro-pillars of organic material (PMMA) to be formed. However, the self-organizing of the PMMA film provides the driving force in the formation of the micro-pillars. For this reason, it has been difficult to control the position, diameter and height of the micro-pillar freely.

SUMMARY OF THE INVENTION:

The present invention provides a method for manufacturing functional elements equipped with a group of columnar micro-pillars of organic polymer material, allowing an easy control of the dimensions and aspect ratio of the micro-pillars. The present method is handy and less expensive.

The method of the invention relates to the manufacturing of functional elements equipped with a group of columnar micro-pillars of organic polymer arranged on a matrix of organic polymer provided on a supporting member in a predetermined pattern, wherein the micro-pillars have an equivalent diameter D of 10 nm to 500 $\mu$m, a height H of 50 nm to 5000 $\mu$m and an aspect ratio H/D of 4 or more,

and wherein the equivalent diameter D of the tip ends of the micro-pillars is smaller than that of the bottom surface; the method comprises the following steps:

- applying a thin film of a thermoplastic resin or of an uncured thermosetting resin on a supporting member,

- pressing a micro-mold comprising a group of pits in the predetermined pattern against the thin film, and
- separating the micro-mold from the thin film, thereby stretching the part of the thin film pressed into the pits to form the group of micro-pillars having an equivalent diameter slightly smaller than the inner diameter of the pits, but having a length greater than the pit depth,
- followed by curing when an uncured thermosetting resin is used.

The functional element equipped with the group of micro-pillars as obtained by the method of the present invention contains a first matrix or supporting member of organic polymer and a group of columnar micro-pillars of organic material extending from this matrix. The micro-pillars have an equivalent diameter of 10 nm to 500 nm and a height of 50 nm to 5000 $\mu$m. The present invention is particularly suited for manufacturing functional elements having a group of micro- pillars in the order of nanometers.

Accordingly, the aforementioned micro-pillars is preferably to have an equivalent diameter of 50 nm or more but less than 1 $\mu$m with a height 100 nm or more but not more than 10 $\mu$m. It should be noted that the functional element obtainable by the method of the present invention includes a film and sheet.

In the present specification, the word "organic polymer" means not only pure organic polymers, but also organic polymer materials containing inorganic or organic fillers or chemically modified materials, unless otherwise specified.

BRIEF DESCRIPTION OF THE DRAWINGS:

Fig. 1 is a perspective view representing a functional element manufactured according to an embodiment of the method of the present invention;
Fig. 2 is a side view representing the structure of a functional element manufactured according to an embodiment of the method of the invention;
Fig. 3 is a side view representing an example of the structure of micro-pillars made according to an embodiment of the method of the invention;
Fig. 4 is a flowchart illustrating the manufacturing method of the present invention;
Fig. 5 is a schematic plan view representing the configuration of an optical circuit as an embodiment of the functional element which can be made in accordance with the present invention;
Fig. 6 is a schematic plan view representing the structure of an optical wave-guide oscillation section in Fig. 5;
Fig. 7 is a perspective exploded view representing the configuration of a micro-biochip;
Fig. 8 is a perspective exploded view representing the configuration of a molecular filter shown in Fig. 7;
Fig. 9 is a plan view showing the structure of a cell culture sheet;
Fig. 10 is a side view showing the culture sheet for cell culture given in Fig. 9;
Fig. 11 is a side view showing the configuration and operation of a water- and oil-repellent sheet made according to the present invention;
Fig. 12 is a side view illustrating the operation of a non-colored coloring sheet made according to the present invention;
Fig. 13 is a schematic side view illustrating the operation of an anti-reflective layer made according to the present invention;
Fig. 14 is a schematic side view illustrating the operation of another example of an anti-reflective layer made according to the present invention;
Fig. 15 is a schematic side view illustrating the operation of a further example of an anti-reflective layer made according to the present invention;
Fig. 16 is a flowchart showing the process for forming a nickel thin film layer on the surface of a columnar micro-pillars made according to the present invention; and
Fig. 17 is a flow chart showing manufacturing of another embodiment of a functional element made according to the invention.

DETAILED DESCRIPTION OF THE INVENTION:

The ratio of the height (H) to the equivalent diameter (D) of the micro-pillars (H/D, aspect ratio) is 4 or more. This is because the aspect ratio of over a certain magnitude allows a desired designing of a functional element, leading to a wider variety of applications.

In the present invention, the equivalent diameter of the columnar micro-pillars means the equivalent diameter at an intermediate position of the height of the micro-pillars. The term "equivalent diameter" is used because the cross section of the micro-pillars is not always circular; it may be elliptical, polygonal or asymmetrical. To include all these shapes, the term "equivalent diameter" is used in the present invention. The aspect ratio (H/D) is to be 4 or more, and is preferred to be 8 to 30. For reasons of structural strength, however, it is preferred to be 100 or less.

To manufacture the film or substrate equipped with a group of micro-pillars according to the present invention, a micro-mold (a precision mold) with concave portions (hereinafter referred to as "pits") having specific arrangements formed thereon is pressed against the thin film made of a thermoplastic resin or of an uncured thermosetting resin, thereby forming a pattern in conform to the aforementioned group of pits of the mold.

The aforementioned mold is made of silicon or quartz, for example. When this mold is separated from the thin film, the thermoplastic resin or uncured thermosetting resin is stretched to form a desired group of micro-pillars. Especially, the height of the micro-pillars can be adjusted by the aspect ratio of the irregularities (pits), and through the position and opening area of the concave portions formed on the mold the position and bottom area of the micro-pillars can be adjusted. This manufacturing method will be described later.

The aforementioned group of micro-pillars is preferred to be of self-supporting type. It is also preferred to be capable of becoming independent of the first matrix. Further, it will be good practice to provide a chemical modification to the tip end or all surfaces of each of the micro-pillars, for example, by providing chemical plating (electroless plating), thereby controlling the reflection factor on the side of the micro-pillars.

In the micro-pillars constituting the group of columnar micro-pillars, the equivalent diameter of the bottom surface is slightly greater than that of the tip end. This is advantageous in ensuring the independence and self-supporting property of the resin-made micro-pillars. The micro-pillars have a portion tapering from the root in contact with the first matrix toward the tip end. It is also preferred that the group of columnar micro-pillars and the first matrix be made of the same material. It is essential that the micro-pillars and the matrix connected thereto are designed in an integral structure.

The group of the micro-pillars can be configured in such a structure that the micro-pillars are closely packed. This makes it possible to configure such an arrangement that individual micro-pillars are less likely to be crushed or to be peeled off from the matrix or the supporting member.

For the process of producing the aforementioned micro-pillars, the organic material can be a thermoplastic high molecular material. Or it can be thermosetting high molecular material. For example, the main component of the micro-pillars can be a substance selected from cyclo-olefin polymers, polymethyl methacrylate, polystyrene, polycarbonates, polyethylene terephthalate (PET), polylactic acid and polypropylene. Alternatively, it can be a photo-cured substance obtained by adding a photosensitive material to these materials. Further, it is also possible to add an antioxidant or a flame retardant to these materials.

When the aforementioned mold is pressed against the thin film of thermoplastic resin and is separated therefrom, the resin pressed into the pits is stretched to form micro-pillars that have an equivalent diameter slightly smaller than the inner diameter of the pits but a length greater than the pit depth. The equivalent diameter and the length of the micro-pillars depend on the type, the physical properties (such as the molecular weight) and the molding conditions (such as pit depth, temperature and molding pressure) of the resin to be used. It is desirable to confirm them in advance by various tests and experiments.

When the thin film of the thermosetting resin is uncured, a precision mold provided with pits having a predetermined arrangement is pressed against it, and is then separated, whereby a group of micro-pillars each having the intended shape is formed. This is then cured by thermosetting or photo curing, thereby getting a functional element substrate characterized by excellent mechanical strength. When curing the thermosetting resin, it is necessary to study the curing temperature and heating profile in order to select such conditions as to avoid melting, flowing or deforming of the resin. It goes without saying that a curing agent or curing accelerating agent can be added to the thermosetting resin.

The method of the present invention provides functional elements equipped with a group of micro-pillars of an organic polymer that allows free control of dimensions, aspect ratio and others. in the present invention, the organic polymer is used to manufacture the group of columnar micro-pillars; it is possible to use a simple production technique of pressing, which provides low-priced functional elements.

It is easy to form micro-pillars of a small aspect ratio. The present invention allows a simple method to be used to produce micro-pillars in the nanometer range having a big aspect ratio of 4 or more. The functional elements nanometer size can be utilized for a great variety of applications. It is also possible to change the distance between the center positions of the micro-pillars (the pitch P) to provide various functions.

DESCRIPTION OF THE PREFERRED EMBODIMENTS:

(First Embodiment)

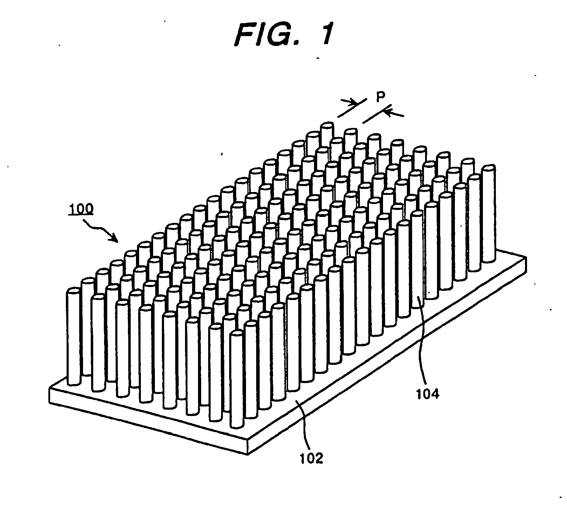

Fig. 1 is a schematic perspective view representing a functional element 100 equipped with a group of micro-pillas 104 manufactured according to the present embodiment. The micro-pillars 104 are made of PMMA, which has a molecular weight of 2,000 to 600,000. The matrix 102 consisting of the same organic polymer as that of the micro-pillars 104 is integral with the micro-pillars and holds the group of micro-pillars firmly in position. In Fig. 1, micro-pillars 104 are arranged in proper alignment both in the longitudinal and transverse directions. They can also be arranged on a random basis. Further, the height and aspect ratio of the group of micro- pillars can be changed in dependence of the position of the micro-pillars. Such changes are one of the characteristics of the manufacturing method of the present invention.

Further, omitting part of the micro-pillars can provide a special function to the functional element. The method according to the present invention allows an easy formation of a functional element having a linear defective portion in one process by forming the defective portion in the micro-pillars in advance using a precision mold.

(Second Embodiment)

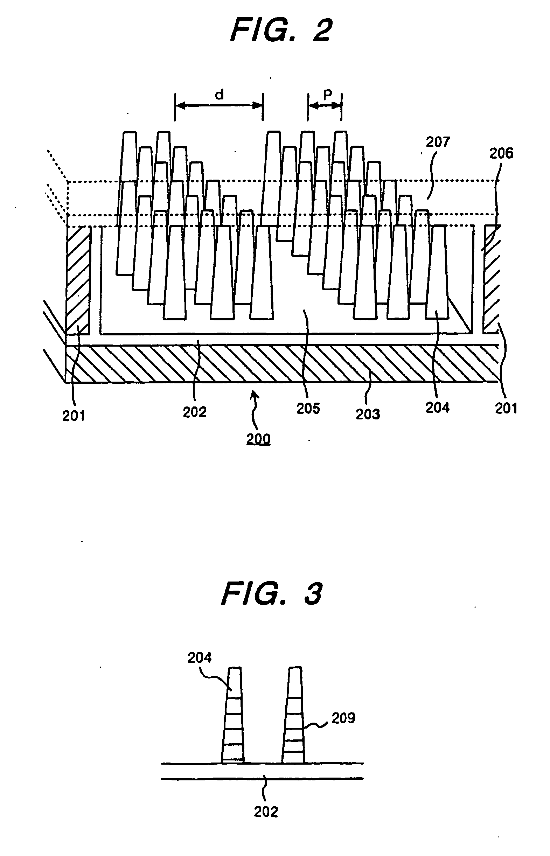

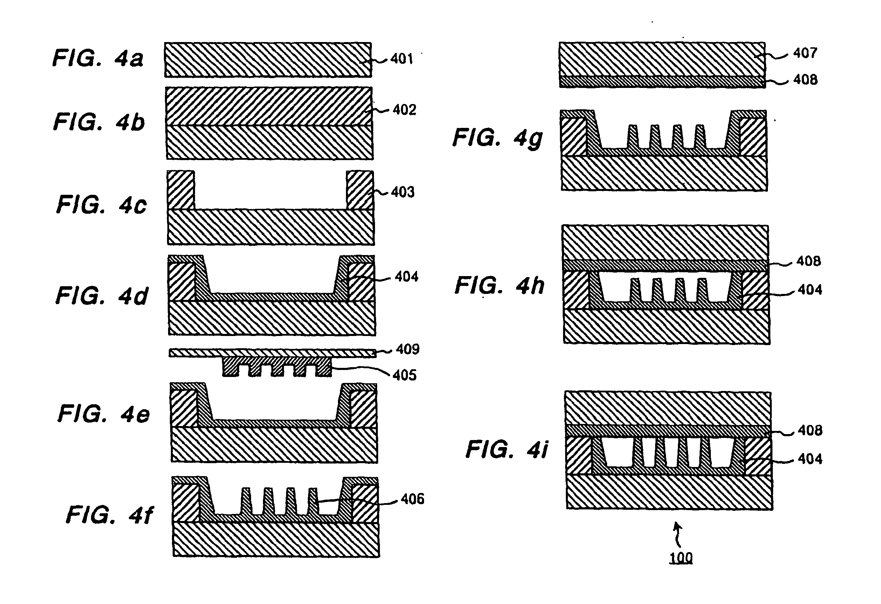

Fig. 2 is a diagram showing the lateral configuration of micro-pillars 204, and Fig. 3 shows a side view of the micro-pillars 204. Fig. 4 is a flowchart representing the process of manufacturing the functionsal element shown given in Figs. 2 and 3.

In Figs. 2 and 3, the height of the columnar micro-pillars 204 is 3 $\mu$m, and the equivalent diameter is 330 nm at the root and 300 nm at an intermediate position. A smooth surface is formed about 1 $\mu$m from the top of the columnar micro-pillars 204, and a lateral striped pattern 209 is formed on the surface of the portion about 2 $\mu$m from the root, as shown in Fig. 3.

The micro-pillars 204 are formed at a certain pitch P, and the equivalent diameter on the bottom surface is 300 nm with a height of 3 $\mu$m, showing that the aspect ratio on the bottom surface is 9. Further, the equivalent diameter of the micro-pillars 204 at the intermediate height is 300 nm, so the aspect ratio at the intermediate position is 10. This is a sufficiently big aspect ratio in excess of 4. The aspect ratio in the sense used in this invention is defined as the value at the intermediate height of micro pillars unless otherwise specified.

It can be seen that each of the columnar micro-pillars 204 has a tip end smaller than the bottom, presenting the flared shape. In this embodiment, the columnar micro-pillars taper from the root toward the tip end. It is also possible to make such arrangements that the micro-pillars taper from the root toward the swelled tip end, for example.

One of the characteristics of the columnar micro-pillars of the present invention is that they have a portion tapering from the root toward the tip end. Since the tip end of the micro-pillars is smaller than the bottom exhibiting the shape of a folding fan, it is difficult to remove the micro-pillars from the supporting member on which the micro pillars are standing. Further, the micro-pillars are made of the same material as that of the underlying supporting member so that the micro-pillars cannot be easily removed from the supporting member.

As will be described later, the columnar micro-pillars 204 are made of PMMA, the same material as that of the underlying film (supporting member) 202. Further, the columnar micro-pillars 204 are connected integrally to the underlying film 202. The portion 205 having a specific width "d" is formed by removing a specific row of the micro-pillar group. This step provides the specific function. A spacer 201 is formed between the first matrix 203 and second matrix 207, thereby protecting the group of micro-pillars. A resin film 206 is formed to protect the tip ends of the micro-pillars and to ensure the close adhesion to the second matrix 207.

Fig. 2 is a perspective view of an optical device 200 for realizing an optical wave-guide manufactured by the method given in Fig. 4. The optical device 200 comprises;

a spacer 201 of silicon nitride having a height of 3 $\mu$m,
a thin film 202 mainly composed of PMMA (with antioxidant and flame retardant added thereto) having a thickness of 0.5 $\mu$m,
a first substrate 203 made of silicon having a thickness of 550 $\mu$m and having a width of 1 mm,
a second substrate 207 made of polycarbonate, and
a group of columnar micro-pillars 204.

The surface of the spacer 201 is covered with a resin film of the same material as the micro-pillars 204 and the film 202.

In the present embodiment, the columnar micro-pillars 204 and the underlying film 202 are made of PMMA, but can also be made of cycloolefin polymers, polystyrenes, polycarbonate, polyethylene terephthalate (PET), polylactic acid, polypropylene, polyethylene, polyvinylalcohol, ABS resins, AS resins, polyamides, polyacetals, polybutylene terephthalate, glass reinforced polyethylene terephthalate, modified polyphenylene ethers, polyvinyl chloride, polyphenylene sulfide, polyetherether ketones, liquid like polymers, fluoride resins, polyacrylates, polyether sulfones, polyamideimides, polyetherimides, thermoplastic polyimides, phenol resins, melamine resins, urea resins, epoxy resins, unsaturated polyester resins, silicone resins, diallylphthalate resins, polyamide bismaleimide resin, polybiamide triazole resins and other resins. Two or more of the materials can be mixed, or photo-activators, oxidation inhibitor agents, flame retardant agents, etc. can be added to the materials.

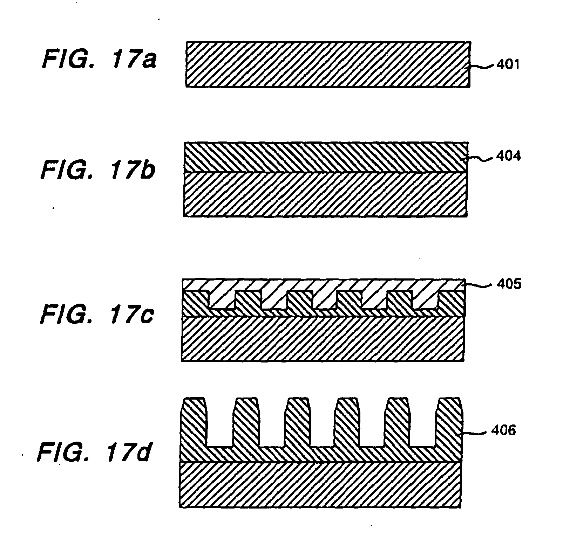

The method of manufacturing the main part of the functional element is shown in Fig. 17. The supporting member 401 is made of a silicon wafer having a crystal orientation of (100) and a diameter of 150 mm. The supporting member 401 was covered with a thin film 404 of PMMA having a molecular weight of 120,000, the film being coated by spin coating . The thickness of the film was 1.5 $\mu$m.

Then, a precision mold 405 having pits on the surface thereof was pressed to the film 404. The mold 405 had a diameter of 150 mm and was a silicon wafer having the same crystal orientation as that of the supporting member 401. The mold 405 was lifted vertically to separate it from the film, thereby forming columnar micro-pillars 406. As shown in Fig. 17, the aspect ratio of the columnar micro- pillars was 4 times that (about 1) of the pits of the mold.

Although it was difficult to form pits of the order of nanometers that have a large aspect ratio, the columnar micro-pillars having a large aspect ratio can be easily manufactured by the method of the present invention.

In the present embodiment, the silicon wafer having a crystal orientation of (100) and a diameter of 150 mm was used as the supporting member 401. However, the substrate 401 is not restricted to a silicon wafer. For example, an inorganic material such as glass, an organic material such as polycarbonate or their laminated structures thereof can be used as the substrate 401.

In the present embodiment, a thin film 404 of PMMA was formed on the surface of the supporting member 401. In addition to the PMMA, cycloolefin polymers, polystyrene, polycarbonates, polypropylene or such organic polymers can be used for this purpose. An inorganic material such as silica can be added to these polymers.

In the present embodiment, a silicon wafer having a crystal orientation of (100) and a diameter of 150 mm was used as the supporting member 401. It is also possible to use a thin film of a metal such as nickel or an organic substance such as PDMS.

Further, the diameter and height of the columnar micro-pillars 406 can be controlled by adjusting the depth of the concave portions (pits) of the mold 405, or the thickness and viscosity of the thin film 404. The size of the bottom of the columnar micro-pillars 406 can be controlled by increasing the opening area of the concave portions of the mold 405. Further, the positions for forming the columnar micro-pillars 406 can be controlled by adjusting the positions of the pits of the mold 405.

Moreover, when a thermoplastic resin is used to make the columnar micro-pillars 406, it is also possible to adjust the temperature of the columnar micro-pillars 406 and to control the shape of the columnar micro pillar 406 easily. Furthermore, if the photo-curable material to form the columnar micro pillar 406 is used, light is applied at the time of forming the columnar micro-pillars 406, thereby ensuring an easy control of the columnar micro-pillars 406.

In the following, the details of the method for manufacturing an optical device 200 are described. Fig. 4 is a flowchart illustrating the method for manufacturing an optical device 200. At first, a silicon nitride film 402 having a thickness of 3 $\mu$m was deposited on the first substrate 401 made of silicon shown in Fig. 4(a) according to the plasma CVD method, as shown in Fig. 4(b).

Then the silicon nitride film 402 was patterned to form a spacer 403 as shown in Fig. 4(c) according to the method of photolithography. Then the first resin film 404 was formed on the spacer 403 and first substrate 401 by spin-coating a PMMA solution (solvent: ethyl cellosolve) added with 5 percent by weight of ultra fine silica particles having a particle diameter of 2 nm, as shown in Fig. 4(d).

Then the mold (precision mold) 405 shown in Fig. 4(e), where the pit having a depth of 1 $\mu$m and a diameter of 500 nm are formed on the surface at a pitch of 1 $\mu$m, is pressed against the first resin film 404, and part of the resin film is pressed into the pit. After that, the group of columnar micro- pillars 406 is formed, as shown in Fig. 4(f), by separating the mold 405. At the back face of the mold 405, a plate 409 is provided to make the pressing force to the film even and horizontally, as well as to keep the film and the face of the mold in parallel with each other.

The shape of the group of columnar micro-pillars is shown in Fig. 2. The micro-pillars 204 are arranged to have a height of 3 $\mu$m and a cycle (pitch P) of 1 $\mu$m. The equivalent diameter is about 300 nm at the intermediate position in the direction of height of the micro-pillars, and 330 nm at the root. As shown in Fig. 3, the portion about 1 $\mu$m on the upper part of the micro-pillars 204 is formed to have a smooth surface, and the surface of the portion about 2 $\mu$m from the root has a striped pattern in some cases.

Further, the equivalent diameter at the intermediate height of the micro-pillars 204 is 300 nm, and the height is 3 $\mu$m, so it is apparent that the ratio of height to one side (aspect ratio) is 10, and is sufficiently greater than 4. It is also apparent that the sectional area of the tip end of the micro-pillars 204 is small, exhibiting the shape of a folding fan. Further, the micro-pillars 204 are made of PMMA, the same material as that of the thin film 202. The micro-pillars 204 are bonded integrally to the thin film 202.

Then the second resin film 408 having the same component as that of the resin film 404 was formed on the second substrate 407 of polycarbonate, as shown in Fig. 4(g) according to the spin coating method. This was followed by the step of laying the second substrate 407 thereon, as shown in Fig. 4(h). After that, a pressure of 10 MPa was applied to the first substrate 401 and the second substrate 407, and heating was provided at 150 degrees Celsius for two minutes, thereby getting an optical device 200 shown in Fig. 4(i). It should be noted that, in the process of Fig. 4(h), the resin film 404 was integrally bonded with the resin film 408.

The micro-pillars 204 were integrally bonded with the thin film 202. The micro-pillars 204 are arranged at a cycle (pitch P) of 1 $\mu$m so that a photonic band gap appears. Further, micro-pillars 204 are not arranged in the path for allowing light to pass through. A gap (optical path) 205 having a width of "d" is formed.

The micro-pillars of the present invention need not always be formed in the shape shown in Fig. 2. When the group of columnar micro-pillars is formed according to the press mold method for thermoplastic resin films to be described below, the approach shown in Figs. 2 and 3 is commonly practiced. To put it another way, the top end of the micro-pillars 204 has a smaller sectional area than the bottom end. As shown in Fig. 3, the top of the micro-pillar is smooth, but the bottom is provided with several lines 209 in the lateral direction in some cases. The reason for generating these lines is not clear, but this is one of the configuration characteristics of the micro-pillars according to the press mold method.

For simplicity, only seven micro-pillars 204 are shown in one row in Fig. 2. In the optical device 200 manufactured on the tentative basis, 80 micro pillars 204 are formed in one row.

Light was applied from the end of the optical device 200 having a length of 30 centimeters, and the spectrum of transmitted light was checked. The result of this test has revealed that light having a wavelength of 1.8 $\mu$m is transmitted. The transmittance was 80 %

In this present embodiment, the main component of the micro-pillars 204 and the thin films 404 and 408 was PMMA. It is also possible to use other thermoplastic resins, for example, high molecular materials including cycloolefin polymers, polystyrene, polycarbonates, polyethylene terephthalate (PET), polylactic acids or polypropylene. Further, the refractive index can be changed by adding ultra-fine particles such as silica and gold to the high molecular material. It is also possible to bond other monomer on the surface of the formed micro-pillars or to provide chemical modification by plating. The wavelength of transmitted light can be adjusted by changing the equivalent diameter and the pitch. That this adjustment is possible is a feature basically different from the prior art described in the aforementioned document 1. Application to communication devices conforming to various communication wavelengths is possible by adjusting the equivalent diameter and pitch of the micro-pillars 204 in conformity with the communication wavelength. Further, photo-cured resins can be used. In this case, the micro-pillars are hardened during press-forming and after molding.

As described above, the micro-pillars 204 of the optical device 200 are formed by press molding. This method does not require dry etching or photolithography, unlike prior art optical devices or semiconductor processes. This method has the advantage that optical devices 200 can be manufactured at lower costs.

Further, with prior art micro-pillars made of silicon root material, the micro-pillars were observed to have been damaged when pressure was applied to the second substrate 407 and first substrate 401 placed in combination as shown in Fig. 4(h). In the present embodiment, however, little damage is observed even when the micro-pillars 406 are brought in contact with the resin film 408, since a high-molecular material is used as the major component of the micro-pillars 204. To put it another way, close adhesion with the resin film 408 and fixing in position are ensured without the micro-pillars 204 being damaged. Further, incoming light does not leak from the gap between the micro-pillars 204 and the second resin film 408. This improvement of adhesion reduces the background noise of the output light.

The optical device requires the micro-pillars to be formed on a periodic basis in order to ensure that the photonic band gap appears. The photonic band gap can be obtained by forming the areas with different refractive indexes at a pitch of half the light wavelength. Accordingly, the wavelength of the transmitted light can be controlled by adjusting the space (pitch) between the micro-pillars. To transmit light of short wavelength on a selective basis, it is necessary to reduce the space between the micro-pillars and to reduce the equivalent diameter or one side of the micro-pillars.

The height of the micro-pillars is related to the aperture for admitting incoming light to be used in the optical device, so this height is preferred to be at least several $\mu$m. The method of manufacturing the micro-pillars in the present embodiment provides the advantage of ensuring a sufficient intensity of incoming light because it allows the aspect ratio of the micro-pillars to be increased.

According to the present embodiment, the tip end of the micro-pillars is smaller than the bottom, showing the shape of a folding fan. This feature makes it difficult to remove the group of micro-pillars from the substrate. Moreover, the group of micro-pillars is made of the same material as the underlying material. This feature also makes it difficult to remove the group of columnar micro-pillars from the underlying material. The optical device can be easily handled because the micro-pillars are integral with the substrate.

(Third Embodiment)

The present embodiment refers to an example of applying the optical device 200 for changing the traveling direction of incoming light, to an optical information processing apparatus. Fig. 5 is a schematic block diagram of the optical circuit 500 manufactured by the present invention. The optical circuit 500 is composed of ten transmission units 502 consisting of indium-based semiconductor lasers and driver circuits, optical wave-guides 503 and 503', and optical connectors 504 and 504' arranged on the substrate 501 of aluminum nitride having a length (1) of 30 mm, a width (w) of 5 mm and a thickness of 1 mm. The transmission wavelengths of the ten semiconductor lasers are each made different in the range from 2 through 50 nm. The optical circuit 500 is a basic component of optical multiplex communication system devices. The present invention is applied to the optical wave-guides 503 and 503'. In the optical wave-guide 503, the optical signal inputted from the transmission unit 502 is received by the oscillation section 503' of the connecting section and is sent to the optical connector 504 from the connecting section 504' through the sub-wave-guide 506 and main wave-guide 505 sequentially. In this case, the optical input signal is formed by merging of sub-wave-guides.

The configuration of the oscillation section 503' is shown in Fig. 6. The connection section 504' is designed in a configuration as a mirror image of the one given in Fig. 5.

Fig. 6 is a schematic layout diagram representing micro-pillar 508 inside the optical wave-guide 503. To permit misalignment between the transmission unit 502 and the optical wave-guide 503, the width of the input section of the optical wave-guide 503' is 20 $\mu$m, and the plane section is configured in a flared form. In the straight central portion, one row of the group of micro-pillars is removed to form an area free of any photonic band gap. This allows the signal light to be led to the area $|w_1)|$ having a width of 1 $\mu$m. It should be noted that the space (pitch) between the micro-pillars 508 is 0.5 $\mu$m. For simplicity, the number of the micro-pillars 508 is smaller in Fig. 6 than the actual number.

The optical circuit 500 makes it possible to output optical signals having ten different types of wavelength in a superimposed form. Since the traveling direction of the light can be changed, the lateral width of the optical circuit 500 can be as small as 5 mm, thereby reducing the size of the device for optical communications. Since the micro pillars 508 can be formed by the press-molding method, the production costs are reduced. The device of the present embodiment superimposes inputted beams of light. It is apparent that the optical wave-guide 503 is useful to all optical devices that control an optical path.

As described in the aforementioned embodiment, the present invention provides optical devices on the order of nanometers that allow free selection of a desired wavelength of the light used as an optical signal, by application of the film equipped with a group of micro-pillars according to the present invention. The present embodiment provides an easy formation of a photonic band gap and micrometer-sized or nanometer-sized optical path when the organic polymer or material mainly composed of organic polymer is used to form the group of micro-pillars of the order of nanometers.

When the aforementioned mold is pressed against the thin film of the thermoplastic resin, and the film is separated, the resin pressed into the pits is stretched, thereby forming a group of micro-pillars whose equivalent diameter at the root is slightly smaller than the pit diameter but having a length greater than the pit depth. The equivalent diameter and length of the micro-pillars depend on the type, the physical properties (molecular weight) and the molding conditions (pit depth, temperature, molding pressure, etc.) of the resin used. Accordingly, it is preferred that various experiments be conducted in advance for verification. In any case, this condition setting determines the conditions for getting the micro-pillars and the optimum photonic crystal, i.e., optical device can be obtained from the relationship with the wavelength of the signal to be used.

The optical device which is obtained by the method of the present invention is characterized as follows: the micro- pillars are arranged according to a predetermined layout method where the organic polymer is used as the main component. An optical path is constituted according to the photonic band gap to input or output the optical signal. Further, the micro-pillars consisting of the organic polymer are arranged in the space sandwiched between two opposing substrates, this substrate being provided according to a predetermined layout method, thereby constituting the photonic band gap. It is preferred that the micro-pillars be connected to the thin film formed on the surface of the substrate. It is especially preferred that the material of the thin film formed on the substrate surface be the same as that of the micro-pillars. A predetermined refractive index can be provided by adding at least one type of material selected from fine particles of oxides, nitrides and metals, to the aforementioned organic polymer. It is also preferred that two opposing substrates and space separating these substrates be provided to support the micro- pillars.

Here the equivalent diameter (diameter or one side) of the micro-pillars can be adjusted freely in the range of 10 nm to 10 $\mu$m, based on the relationship with the wavelength of the light source used for the semiconductor laser or the like. The height of the micro-pillars is preferred to be 50 nm 10 $\mu$m. The distance (pitch) between the micro pillars is about half the wavelength of the signal to be used.

(Fourth Embodiment)

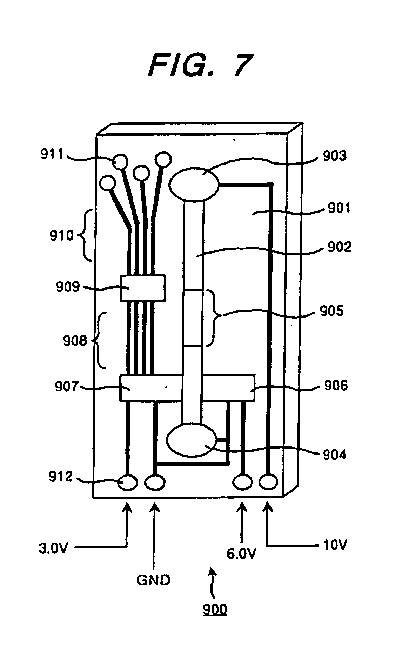

In the present embodiment, the micro- pillar assembly 100 shown in the first embodiment is applied to a biochip 900. Fig. 7 is a schematic plan view representing the biochip 900. A flow path having a depth of 3 $\mu$m and a width of 20 $\mu$m is formed in the glass-made substrate 901. A sample containing DNA (deoxyribonucleic acid), blood, protein and others is led into the inlet 903, and is sent towards the outlet 904 through the flow path 902.

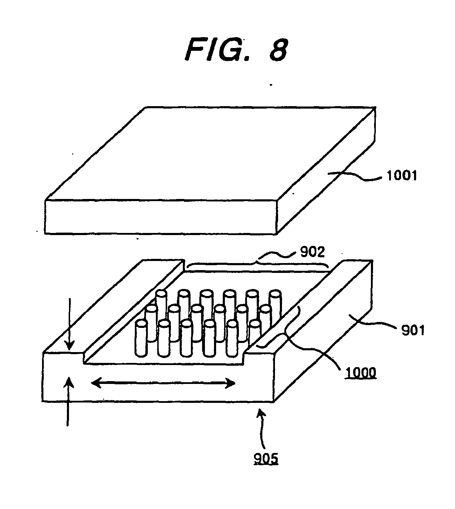

A molecular filter 905 is installed in the flow path 902. A micro-pillar group 1000 (Fig. 8) having a diameter of 250 to 300 nm and a height of 3 $\mu$m is arranged in the molecular filter 905. Fig. 8 is a perspective view of the molecular filter 905. A flow path 902 is formed on the substrate 901. The micro pillar group 1000 is formed in a part of the flow path 902.

The substrate 901 is covered by the upper substrate 1001, and the sample moves inside the flow path 902. In the analysis of a DNA chain length, the DNA-containing sample is separated according to the DNA chain length when passing through the molecular filter 905 in the flow path 902 by electrophoresis. To put it another way, DNA of shorter chain length passes through the molecular filter faster than DNA of longer chain. This allows the DNA to be separated according to the chain length.

From the semiconductor laser 906 mounted on the surface of the substrate 901, laser light is applied to the sample having passed through the molecular filter 905. When DNA passes by, the incoming light to the optical detector 907 is reduced by about 4 percent. This allows the DNA chain length to be analyzed using the output signal from the optical detector 907.

The signal detected by the optical detector 907 is inputted into the signal processing chip 909 through the signal wiring 908. The signal processing chip 909 is connected to signal wirings 910, which are connected with output pads 911. connected to an external terminal. The power is supplied to each component from the power supply pad 912 installed on the surface of the substrate 901.

The molecular filter 905 in the present embodiment is composed of the substrate 901 equipped with a concave portion, wherein many micro-pillars are formed on the concave portion of the substrate 901 and an upper substrate 1001 formed to cover the concave portion of the substrate 901. Here the tip ends of the micro-pillarsare formed so as to contact the upper substrate.

An organic polymer is the major component of the micro-pillar group 1000, which can be deformed. This protects the upper substrate 1001 against damage when the flow path 902 is covered with the upper substrate 1001, and allows the upper substrate 1001 to come in close contact with the micro-pillar group 1000. This configuration prevents the sample from leaking through the gap between the micro-pillar group 1000 and the upper substrate 1001, thereby ensuring a highly sensitive analysis.

When the DNA was subjected to chain length analysis, the resolution of the base pairs was ten base pairs in terms of half-width in the case of a glass-made micro-pillar group; whereas the resolution of the base pairs was three base pairs in terms of half-width in the case of a micro-pillar assembly 1000 of organic polymer. This test has revealed that such an improvement can be achieved. In the molecular filter of the present embodiment, the micro-pillar group is brought into direct contact with the upper substrate. It is also possible to make such arrangements that the film of the same material as that of the micro- pillars is formed on the upper substrate, and the micro-pillars and this film are kept in contact with each other, for example. This arrangement improves adhesion.

In the present embodiment, only one flow path 902 is used. Simultaneous multiple analyses can be made by forming a plurality of flow paths 902 by providing micro-pillars of different sizes. In the present embodiment, a DNA is used as a sample for analysis. If the surface of the micro-pillars of the micro-pillars group 1000 is modified with a molecule reacting with a sugar chain, a protein or an antigen in advance, it is possible to analyze the sugar chain, the protein or the antigen. In this manner, the sensitivity of immunoanalysis can be improved by modifying the surface of the micro-pillars.

The present embodiment allows a simple formation of micro-pillars of organic polymer for analysis on the order of nanometers. It also permits the position, diameter, height and aspect ratio of the micro-pillars of organic material to be controlled by adjusting the irregularities on the mold surface and the viscosity of the thin film of the organic material. It further provides a micro-biochip of high sensitivity for analysis.

In the micro-biochip in the present embodiment, it is preferred that multiple micro pillars be arranged on the sample detection section, these micro pillars be made of organic polymer, and the diameter of these micro-pillars be 10 nm to 100 $\mu$m with a height of 0.5 to 500 $\mu$m. The diameter or one side of the micro- pillars is preferred to be 10 nm to 100 $\mu$m, the same size as that of biopolymers or cells. The height of the micro-pillars is adjusted to the height of the microchip flow path. A microchip of high sensitivity for analysis can be created by interaction ofa biopolymer (especially biopolymer) with a cell if the surface area for reaction is increased by providing multiple micro-pillars on the detecting section of the microchip.

In the micro-pillar in the present embodiment, the ratio of the height to the diameter or one side is sufficiently greater than 1; therefore, micro- pillars can be arranged in the microscopic flow path having a depth of several micrometers or more.

(Fifth Embodiment) (Cell Culture Sheet)

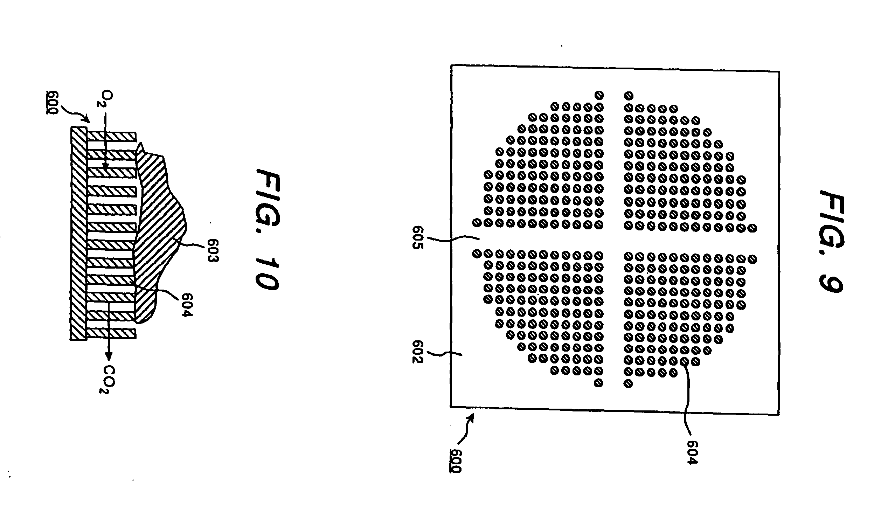

Fig. 9 is a plan view representing a cell culture sheet 600. The cell culture sheet 600 is a thin film 602 mainly composed of a 0.5 $\mu$m-thick PMMA, and a group of columnar micro pillars 604 having an equivalent diameter of 2 $\mu$m mainly composed of a 0.5 $\mu$m-thick PMMA, where the group of micro- pillars 604 extends from the thin film.

A gap 605 is formed by removing a part of the group of micro-pillars. This cell culture sheet is placed in a glass-made Petri dish of an other vessel, in which culture solution is placed. As shown in Fig. 10, cells or the like are cultured by placing such cells (tissue) as of skin, bone or blood, culturing media and culture solution 603 including nutrient on the micro-pillars 604 of the cell culture sheet 600.

It is preferred that the group of micro- pillars be provided with a gap 605 of a certain size formed by removing part of the micro-pillars. In the present embodiment, gaps are arranged in a cross form, as shown in Fig. 9. Formation of such gaps ensures an easy flow of the culture solution and an effective supply of nutrient to the cells. It also permits an efficient removal of waste material from the cells at the time of cell culture.

It has been proven that the use of this cell culture sheet substantially reduces the damage of the sheet-like epidermal cells caused by separation from the Petri dish when a conventional glass Petri dish is used. Further, it has been possible to improve the plating efficiency in the transplantation of sheet-like epidermal cells onto rabbit skin. The culture solution can easily flow to the entire sheet-like epidermal cells through the gap formed on the bottom of the epidermal cells created by the micro-pillars on the cell culture sheet, thereby ensuring an efficient supply of nutrient to the cells or an efficient removal of waste material from the cells. This has resulted in reducing the death of epidermal cells during cell culture that has often occurred so far.

The following describes how to produce the cell culture sheet: The PMMA solution (solvent: ethyl cellosolve) is applied on the mold side of the stage of a press device, thereby forming a resin film. A mold with a great number of pits each having a depth of 1 $\mu$m and a diameter of 500 nm at a pitch of 1 $\mu$m formed on the surface, is pressed against the resin film, and part of the resin film is pressed into the pit. Then the mold is separated to form a group of micro- pillars so as to get a cell culture sheet with a group of micro-pillars formed on the thin film. In the present embodiment, the resin film is applied directly on the stage. It is also possible to form the cell culture sheet by applying the resin film on a substrate of silicon or the like.

The group of micro pillars has a height of 3 $\mu$m, and the micro-pillars are arranged at a pitch of 1 $\mu$m. The equivalent diameter of the micro- pillars is 300 nm at the intermediate position in the direction of height and is 330 nm at the root. Accordingly, the aspect ratio of the micro-pillars is 10. The portion of about 1 $\mu$m on the upper part of the columnar micro pillar is formed to have a smooth surface, and the surface of the portion of about 2 $\mu$m from the root has a striped pattern in some cases. Further, the sectional area of the tip end of the micro-pillars is smaller than that of the bottom, showing the shape of a folding fan.

The cell culture sheet formed in the present embodiment was placed in the glass-made Petri dish with culture solution therein, and normal human epidermal cornified cells (used medium: HuMedia-KB2 (by Kurabo Inc.) at 37°C under the flow of 5% $CO_2$) were cultured on the cell culture sheet according to the conventional method. In this experiment, epidermal cornified cells were correctly attached to the cell culture sheet and proliferated in the form of a sheet.

On the fourteenth day after starting the culture, the cultured cells were covered with a polyvinylidene difluoride (PVDF) film having a diameter of 2 cm, and the medium was drawn by suction, whereby the sheet-like epidermal cornified cells grown on the nano-pillar sheet were separated from the cell culture sheet together with the PVDF film. These sheet-like epidermal cornified cells could be removed from the covering PVDF film easily. Damage to the sheet-like epidermal cornified cells caused by separation from the cell culture sheet could be substantially reduced as compared to the case where the conventional glass-made Petri dish was used.

It is also possible to make the high molecular material hydrophilic by plasma treatment. The high molecular material is not restricted to a particular type, but it is preferred that the selected material does not exert a serious effect to the cells (tissue) to be cultured. For example, selection of polystyrene, PMMA, or polylactic acid is preferred.

In the present embodiment, the micro pillars have a tip end smaller than the bottom, presenting the shape of a folding fan. This configuration prevents the micro- pillars from being removed from the substrate. Since the micro-pillars are made of the same material as the underlying material, the group of micro-pillars is not easily removed from the substrate. Further, handling of the cell culture sheet is facilitated by the integral configuration of the micro-pillars with the substrate.

(Sixth Embodiment) (water repellent sheet)

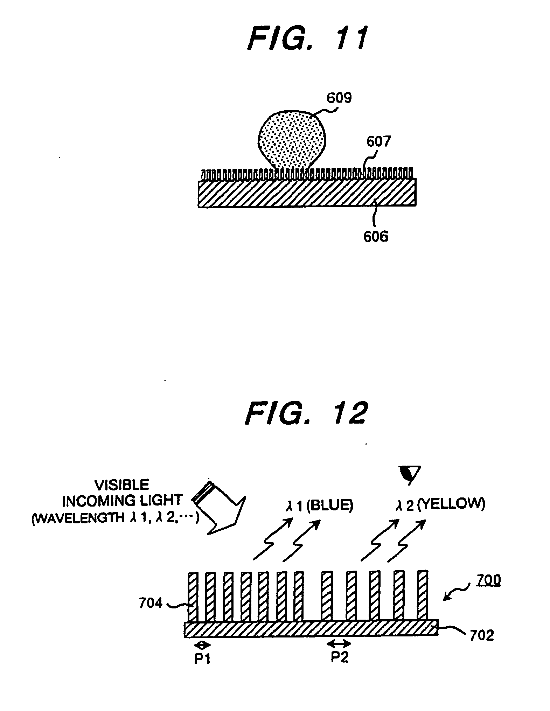

In the following, a further embodiment of the present invention is described. The present embodiment shows the case of using a thin film (sheet) as a water-repellent film, wherein the thin film comprises as a group of micro-pillars formed on its surface. As shown in Fig. 11, the water-repellent film 605 of the present embodiment consists of micro-pillars 607 formed on the sheet 606. In the present embodiment, it is preferred that the pitch (gap) between the micro-pillars be from 20 nm to 10 $\mu$m with the height ranging from several $\mu$m through several tens of $\mu$m, and the diameter of the tip end of the micro- pillars be 50 to 500 nm. The material of the micro-pillars is not restricted to a particular type, but it is preferred to use a water-repellent material such as PMMA. Alternatively, it is also possible to treat the surface of the micro-pillars with a water-repellent agent.

With the water-repellent film of the present embodiment, the contact angle of a water drop 609 can be increased by the micro- pillars 607 on the surface, as compared to the case of using a water-repellent sheet 606 as a single body, as shown in Fig. 11, and the water repellent property of the material can be increased by the "lotus leaf effect" obtained by the group of micro-pillars 607. Since the water-repellent group of micro-pillars of the present embodiment can be arranged in a closely packed configuration on the order of nanometers, it is not easily crushed or removed. It is also characterized by persistent water- and oil-repellency.

The water- and oil-repellent film of the present embodiment can be applied to the surface of an umbrella, clothing and a wall, for example. In this case, a water-repellent film is produced as follows: A sheet is manufactured according to the same method as that used in the fifth embodiment, wherein this sheet has micro- pillars formed thereon.

Then this sheet is laminated using a pressure sensitive adhesive. Another method is to transfer the sheet directly on the surface of the matrix of the umbrella, clothing or wall where water- and oil-repellent oil film is to be formed.

The water-repellent film of the present embodiment eliminates the need of a water-repellent treatment that has been commonly used in the prior art method. It provides a simple modification of the material surface in one transfer operation.

(Seventh Embodiment) (Color sheet free of coloring agents)

In the following, a seventh embodiment is described where a group of micro-pillars formed on a sheet is used as a color sheet free of coloring agents or a non-dyed color producing sheet. Fig. 12 shows the color producing principle of the color sheet free of coloring agents. Visible light entering the micro-pillars is reflected while causing interference in the group of micro-pillars. This appears as if the sheet surface were colored with a coloring agent. As shown in Fig. 12, the interference of the visible light can be varied by changing the spaces (pitches) P1 and P2 between the micro-pillars, with the result that the visible color tone is changed.

For example, when white light containing the wavelength components $\lambda 1, \lambda 2$ ... is applied to the group of micro-pillars 704 of the sheet 700, the light reflected from areas with smaller pitch "P1" is turned into light of $\lambda 1$ (blue), while the light reflected from areas of greater pitch "P2" is turned into light of $\lambda 2$ (yellow).

As the space between the micro-pillars is increased, the interference color having a greater wavelength is intensified in such a way that the color is changed in the order of blue, green, yellow and red. Adjusting the space between the micro-pillars in this manner allows the sheet to appear as if it were colored, without using a dye or pigment. It is also possible to change the color in dependence of the place. Further, if coherent light is used the coloring sheet free of coloring agent changes the color tone according to the angle of viewing.

In a similar manner, the interference of visible light can be changed by adjusting the diameter and height of the micro pillars. The color tone of the sheet can be changed by adjusting the diameter and height of the micro pillars.

In the non-dyed coloring sheet of the present embodiment, to ensure that the interference of visible light will occur, the diameter of the micro- pillars to be used is preferred to be 100 to 2000 nm, and the space between the micro-pillars is preferred to be 100 to 2000 nm, with the height of the micro- pillars ranging from 1 $\mu$m to 5 $\mu$m.

The non-dyed coloring sheet finds application in clothing, on-board sheets, accessories, wall paper, holography, etc.

(Eighth Embodiment) (Anti-reflective layer)

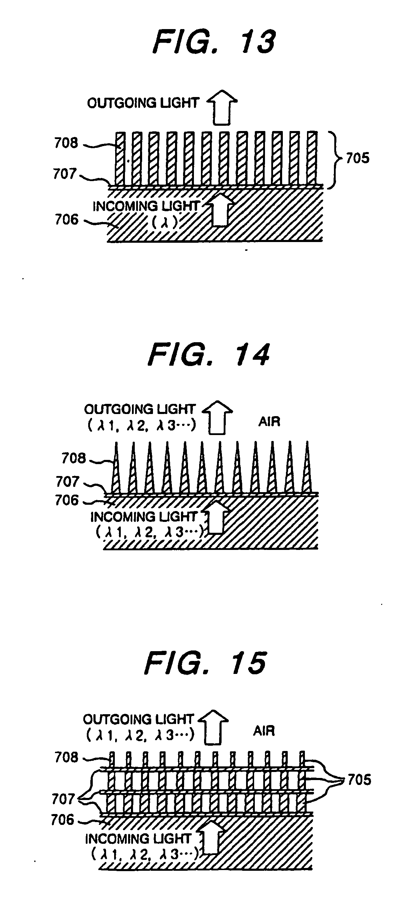

In the following, an eighth embodiment is described where a group of micro-pillars is used as an anti-reflective layer. As shown in Fig. 13, the anti-reflective layer 705 comprises a thin film (sheet) 707 mainly composed of PMMA and having a thickness of 0.1 $\mu$m or less, and a group of micro pillars 708 with an equivalent diameter of 1 $\mu$m or less, mainly composed of PMMA, extending from the thin film 707. This anti-reflective film is formed on the surfaces of display cover glasses and optical substrates for optical communication, and is used as an anti-reflective layer. The anti-reflective layer for display cover glasses and optical substrates for optical communications is manufactured in two ways; by using a pressure sensitive adhesive to laminate the sheet with a group of micro- pillars formed on a thin film, and by applying a resin directly on the substrate such as the cover glass or an optical substrate and forming micro- pillars according to the transfer method, thereby creating an anti-reflective layer. When the sheet is laminated using the pressure sensitive adhesive , the pressure sensitive adhesive to be used is preferred to be a material characterized by excellent optical transparency, such as an acryl based adhesive.

For the anti-reflective layer of the present embodiment, the effective refractive index in the layer composed of the micro-pillars (hereinafter referred to as "reflective index") can be adjusted, as desired, by adjusting the diameter, pitch, height and sectional form of the micro-pillars, and changing the ratio between the dielectric area of the micro-pillars in the layer composed of the micro-pillars and the air area free of any micro pillar. This allows suppression of the light reflected on the surface of the substrate.

The ideal conditions for the refractive index ($n_{AR}$) and thickness ($t_{AR}$) for this anti-reflective layer can be represented by the following formulae:

$$\text{Reflective index: } n_{AR} = (n_{sub} \cdot n_{air})^{1/2},$$

$$\text{Thickness: } t_{AR} = \lambda/(4n_{AR}),$$

where "$n_{sub}$" denotes the refractive index of substrate, "$n_{air}$" the refractive index of air, and "$\lambda$" denotes the designed wavelength.

For example, when the refractive index of the substrate (glass is assumed) is 1.5 and $\lambda$ is 1.5 μm, the refractive index of the anti-reflective layer is 1.22 and the thickness is about 320 nm, according to the above formula. In this way, the parameter required for the anti-reflective layer is calculated and the shape and arrangement of the micro pillars are adjusted, thereby creating the anti-reflective layer of the display cover glass and optical substrate for communication applications.

In the following the structure and functions given in Fig. 13 are described. The light emitting devices are arranged on the side of the substrate 706. The anti-reflective layer 705 is provided to ensure that the light coming from the substrate 706 has reduced reflected light produced on the boundary between the substrate 706 and air. The operating principle of the anti-reflective layer 705 will be described below: In the first place, there is a difference in the refractive index on the boundary between the substrate 706 and the anti-reflective layer 705, so that reflected light #1 which goes back to the substrate 706 is produced. Further, reflected light is emitted also on the interface of the anti-reflective layer 705 and air, and the reflected light undergoes repeated multiple reflections in the anti-reflective layer 705 and transmission from the anti-reflective layer 705. After that, reflected light #2 to return to the substrate 706 is formed. In this case, when the amplitudes of the reflected light #1 and #2 are equal to each other and their phases are opposite to each other, the two types of reflected light cancel each other, with the result that the reflected light to return to the substrate 705 is lost.

For transmitted light on the air side, a transmittance of 100 % can be obtained when transmitted light #1 having passed directly through the anti-reflective layer 705 is in the same phase with the transmitted light #2 formed after repeated multiple reflection in the anti-reflective layer 705 and transmission from the anti-reflective layer 705.

The conditions of the anti-reflective layer for meeting the aforementioned reflection conditions (full transmission conditions) can be represented by the aforementioned formula. Even when light has launched from the side of air, it is possible to get the same anti-reflective effect as that when light is applied from the substrate 706. The configuration given in Fig. 13 indicates an anti-reflective layer using the phase interference of the light, so the anti-reflective effect will be reduced if the incoming light is deviating from the design wavelength.

In the following the structures given in Figs. 14 and 15 are described. They are different from the structure of Fig. 13 in the following points: It is possible to remove the discontinuous surface resulting from the difference in refractive index and to reduce reflection by slowly changing the refractive index in the anti-reflective layer 705 from the refractive index of the substrate 705 to that of air. Since the phase interference of light is not used, the dependence of the refractive index on the wavelength can be reduced.

According to the present embodiment, the area of air in the layer formed by the group of micro-pillars is expanded to realize a layer having a refractive index of as low as 1.3 or less. For example, it is possible to constitute a single-level anti-reflective layer for reducing the light reflected from the surface of the glass substrate having a refractive index of about 1.5. To put it more specifically, when the prior art continuous $MgF_2$ film is used, the refractive index is about 1.3 when it is small. In contrast thereto, in the anti-reflective layer of the present embodiment, the refractive index can be adjusted to about 1.2--a value close to the ideal value--by optimizing the diameter and space of the micro-pillars.

Fig. 14 will be used to describe another type of the anti-reflective layer of the present embodiment. In this example, the micro-pillars of the group of columnar micro pillars 708 are designed as tapering from the root toward the tip end. As a result of this structure, in the range of wavelengths from 300 nm 700 nm, the refractive index is reduced to 1/50 or less of that when there is no anti-reflective layer. As described above, the tapered micro- pillars allow the refractive index in the layer to be changed in the direction of depth, thereby ensuring an excellent anti-reflective effect in a wide wavelength band.

Fig. 15 is used to describe a further type of anti-reflective layer. In this example, sheets 705 with a group of micro-pillars 708 having different refractive indexes are laminated on the supporting member. Here the sheets 705 can be laminated as follows: Single-layer sheets can be laminated using a pressure sensitive adhesive. Alternatively, single-layer sheets can be laminated using compression (e.g. a load of 300 kg/cm$^2$with heating at 90°C).

Similarly to the tapered configuration given in Fig. 14, the refractive index in the direction of depth can be changed by laminating the sheets having different effective refractive indexes as in the present embodiment. This provides an anti-reflective effect in a wide wavelength band.

In addition to the embodiments described above, single-level anti-reflective layers having different wavelength characteristics on the same substrate can be arranged in a desired area by changing the diameter and space of the micro-pillars for each specific area on the substrate.

(Ninth Embodiment) (Highly-sensitive electrode)

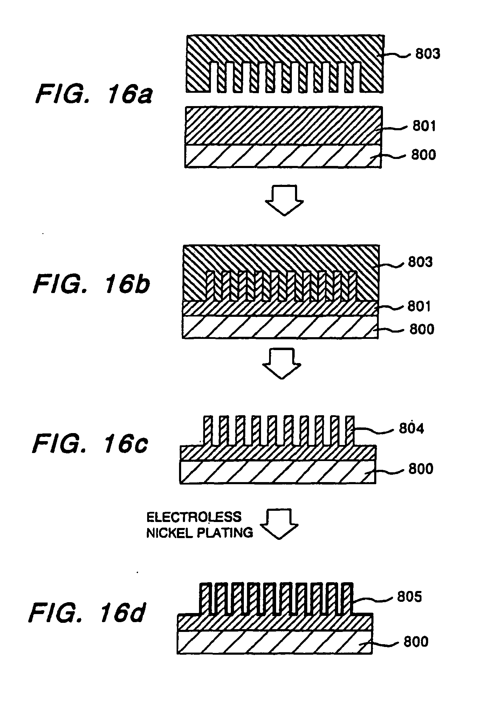

In the following Fig. 16 is used to describe the ninth embodiment where a nickel thin film layer is formed on the surfaces of the micro-pillars through electroless plating, and this is used as a highly sensitive electrode in the anode stripping method, which is a technique for analyzing trace quantities of metal ions.

In the first place, a polystyrene resin layer 801 was formed on a glass substrate having a length of 30 mm, a width of 30 mm and a thickness of 1 mm, using the same method as disclosed in the first embodiment. Then a precision mold 803 was used to produce a substrate having micro-pillars 804 of polystyrene resin on the surface of the polystyrene resin layer 801. The micro-pillars 804 formed in this step had an equivalent diameter of 240 nm, a top diameter of 200 nm and a height of 1 $\mu$m, where the center-to-center pitch of the micro- pillars 804 was 500 nm.

Then the surface of the substrate having micro- pillars of polystyrene on the surface was coated with a 20 nm thick nickel layer by electroless plating. In the following, the electroless plating method used in the present embodiment is described. In the first place, palladium as a nucleus for electroless plating deposition was attached onto the surface of the substrate having the micro-pillars of polystyrene, using the processing solution Neogant of Atotech Japan Inc. This process is generally called "catalytic activation process". The obtained substrate was immersed in the electroless nickel plating solution of Top Chemialloy 66 of Okuno Seiyaku Inc. for 3 minutes so that a nickel layer was formed on the surface of the substrate having micro-pillars on the surface. The obtained substrate surface had a metallic luster. It was observed by a scanning electron microscope to verify that the top diameter of the micro-pillars was 240 nm, and the bottom diameter was 280 nm, with a 20 nm-thick nickel layer uniformly formed on the surface of the micro- pillars.

In the following, the use of the anode stripping process to analyze trace quantities of copper ions contained in an aqueous solution is described wherein the substrate having on the surface the micro-pillars coated with a nickel layer obtained according to the aforementioned method is used as an electrode. The anode stripping method provides a technique of reducing and concentrating on the electrode the metal ion contained in the solution, and applying anode polarization to the electrode to cause reduction and concentration, thereby identifying the ion in solution based on the potential and the flowing electric energy at that time. In the first place, 4 types of copper sulfate solutions were prepared, where the copper ion concentrations were $1 \times 10^{-8}$ mol/l, $1 \times 10^{-9}$ mol/l, $1 \times 10^{-10}$ mol/l and $1 \times 10^{-11}$ mol/l, using ultra pure water.

This step was followed by the measurement of the aqueous solutions of copper sulfate conducted according to the anode stripping method, using;

a substrate having on the surface the micro-pillars coated with a nickel layer, and

a substrate as a comparative example, without micro-pillars on the surface thereof coated with nickel layer, prepared according to the aforementioned method and technique, except for the process for forming micro-pillars. In the measurement, a glass beaker was used and the electrode size was 5 mm$^2$ . Further, a platinum electrode was used as the counter electrode, and a saturated electrode (SCE) as a reference electrode. 30 ml of the aqueous solution of copper sulfate were poured into the beaker. After the electrode, the counter electrode and the reference electrode were immersed, purging was carried out with argon gas.

This was followed by the step of applying a potential of -0.4 V Vs. The SCE for fifteen minutes to the electrode so that copper was deposited on the electrode surface. Then the potential of the electrode was swept at 0.1 V/min from -0.4 V vs. SCE to +1.0 V vs. SCE, thereby dissolving the copper deposited on the surface of the electrode. The potential and current at that time were recorded. The obtained results are shown in Table. The ratio of electric charge for dissolving the copper of the plate electrode (electrode without micro-pillars), to the electric charge for dissolving the copper of the highly sensitive electrode substrate (electrode having micro-pillars) as made according to the present invention was 10 to 15 independently of the copper ion concentration. It has been revealed that measurements can be made with higher precision and sensitivity when the electrode with micro-pillars is used, than when the electrode without micro-pillars is used.

This can be explained as follows: Formation of micro-pillars on the surface of the electrode provides a drastic increase in the surface area of the electrode substrate in the present invention over the smooth electrode (electrode without micro- pillars), with the result that there is an increase in the amount of copper deposited on the surface of the electrode.

Table: Measurements by anode stripping method

| Copper ion concentration | Electric charge $Q_1$ of highly sensitive electrode for dissolving copper /Electric charge $Q_2$ of a the smooth electrode for dissolving copper |
|---|---|
| $1 \times 10^{-8}$ mol/l | 10.5 |
| $1 \times 10^{-9}$ mol/l | 11.0 |
| $1 \times 10^{-10}$ mol/l | 13.5 |
| $1 \times 10^{-11}$ mol/l | 14.8 |

**Claims**

1.  Method for manufacturing a functional element (100) equipped with a group of columnar micro-pillars (104; 204) of organic polymer arranged on a matrix (102; 202) of organic polymer provided on a supporting member (401) in a predetermined pattern, wherein the micro-pillars (104) have an equivalent diameter (D) of 10 nm to 500 μm, a height (H) of 50 nm to 5000 μm and an aspect ratio (H/D) of 4 or more,
    and wherein the equivalent diameter (D) of the tip ends of the micro-pillars (104; 204) is smaller than that of the bottom surface, comprising the following steps:

    - applying a thin film (404) of a thermoplastic resin or of an uncured thermosetting resin on a supporting member (401),
    - pressing a micro-mold (405) comprising a group of pits in the predetermined pattern against the thin film (404), and
    - separating the micro-mold (405) from the thin film (404), thereby stretching the part of the thin film (404) pressed into the pits to form the group of micro-pillars (104; 204) having an equivalent diameter slightly smaller than the inner diameter of the pits, but having a length greater than the pit depth,
    - followed by curing when an uncured thermosetting resin is used.

2.  Method according to claim 1,
    **characterized in that** the aspect ratio (H/D) of the micro-pillars (104; 204) is made to be in the range of 4 to 100.

3.  Method according to claim 1 or 2,
    **characterized in that** the aspect ratio (H/D) of the micro-pillars (104; 204) is made to be in the range of 8 to 30.

4.  Method according to any of claims 1 to 3,
    **characterized in that** the micro-pillars (104; 204) are made to have a portion tapering from the root in contact with the matrix (102; 202) toward the tip end.

5.  Method according to any of claims 1 to 4,
    **characterized in that** at least a part of the micro-pillars (104; 204) has at its tip portion an expanded portion in the diameter direction.

6.  Method according to any of claims 1 to 5,
    **characterized in that** at least the surface of the micro-pillars (104; 204) is made of a substance mainly composed of a water-repellent and/or oil-repellent organic polymer.

7.  Method according to any of claims 1 to 6,
    **characterized in that** at least a part of the surface of the micro-pillars (104; 204) is provided with a metal plating.

8.  Method according to any of claims 1 to 7,
    **characterized in that** the group of micro-pillars (104; 204) comprises clearances (205) having a predetermined spacing.

9.  Method according to any of claims 1 to 8,
    **characterized in that** the micro-pillars (104; 204) are made to have an equivalent diameter (D) of 50 nm to 1 μm and a height of 100 nm to 10 μm.

10. Method according to any of claims 1 to 9,
    **characterized in that** the functional element (100) is made as a film or sheet.

11. Method according to any of claims 1 to 10,
    **characterized in that** the micro-pillars (104; 204) and the underlying matrix (102; 202) are made from a material, the main component of which is selected from polymethyl methacrylate (PMMA), cyclo-olefin polymers, polystyrene, polycarbonate, polyethylene terephthalate (PET), polylactic acid, polypropylene, polyethylene, polyvinylalcohol, ABS resin, AS resin, polyamide, polyacetal, polybutylene terephthalate, glass reinforced polyethylene terephthalate, modified polyphenylene ether, polyvinyl chloride, polyphenylene sulfide, polyetherether ketone, liquid like polymers, fluoro resins, polyacrylate, polyether sulfone, polyamideimide, polyetherimide, thermoplastic polyimide, phenol resins, melamine resins, urea resins, epoxy resins, unsaturated polyester resins, silicone resins , diallylphthalate resins, polyamide bismaleimide resins and polybiamide triazole resins.


**Patentansprüche**

1. Verfahren zur Herstellung eines funktionellen Elements (100), das mit einer Gruppe kolumnarer Mikrosäulen (104; 204) aus einem organischen Polymer versehen ist, die in einem vorgegebenen Muster auf einer Matrix (102; 202) aus einem organischen Polymer angeordnet sind, die auf einem Trägerelement (401) vorgesehen ist, wobei die Mikrosäulen (104) einen äquivalenten Durchmesser (D) von 10 nm bis 500 $\mu$m, eine Höhe (H) von 50 nm bis 5000 $\mu$m und ein Längenverhältnis (H/D) von 4 oder mehr aufweisen und wobei der äquivalente Durchmesser (D) der Spitzenenden der Mikrosäulen (104; 204) kleiner ist als der äquivalente Durchmesser der Bodenfläche, das folgende Schritte umfasst:

   - Aufbringen einer dünnen Schicht (404) aus einem thermoplastischen Harz oder einem nichtgehärteten wärmehärtbaren Harz auf einem Trägerelement (401),
   - Pressen einer Mikroform (405), die eine Gruppe von Vertiefungen in dem vorgegebenen Muster aufweist, gegen die dünne Schicht (404) und
   - Trennen der Mikroform (405) von der dünnen Schicht (404), wodurch der Teil der dünnen Schicht (404), der in die Vertiefungen gepresst wurde, gestreckt wird und die Gruppe von Mikrosäulen (104; 204) bildet, die einen äquivalenten Durchmesser, der etwas kleiner ist als der Innendurchmesser der Vertiefungen, jedoch eine Länge aufweisen, die größer ist als die Tiefe der Vertiefungen,
   - sowie anschließendes Härten, wenn ein nichtgehärtetes wärmehärtbares Harz verwendet wird.

2. Verfahren nach Anspruch 1,
   **dadurch gekennzeichnet, dass** das Längenverhältnis (H/D) der Mikrosäulen 104; 204) so erzeugt ist, dass es im Bereich von 4 bis 100 liegt.

3. Verfahren nach Anspruch 1 oder 2,
   **dadurch gekennzeichnet, dass** das Längenverhältnis (H/D) der Mikrosäulen (104; 204) so erzeugt ist, dass es im Bereich von 8 bis 30 liegt.

4. Verfahren nach einem der Ansprüche 1 bis 3,
   **dadurch gekennzeichnet, dass** die Mikrosäulen (104; 204) so ausgebildet sind, dass sie einen Bereich aufweisen, der sich vom Fuß, der mit der Matrix (102; 203) in Kontakt steht, zum Spitzenende hin verjüngt.

5. Verfahren nach einem der Ansprüche 1 bis 4,
   **dadurch gekennzeichnet, dass** mindestens ein Teil der Mikrosäulen (104; 204) an ihren Spitzenbereich einen in Richtung des Durchmessers verbreiterten Bereich aufweist.

6. Verfahren nach einem der Ansprüche 1 bis 5,
   **dadurch gekennzeichnet, dass** zumindest die Oberfläche der Mikrosäulen (104; 204) aus einer Substanz hergestellt ist, die hauptsächlich aus einem wasserabweisenden und/oder ölabweisenden organischen Polymer besteht.

7. Verfahren nach einem der Ansprüche 1 bis 6,
   **dadurch gekennzeichnet, dass** mindestens ein Teil der Oberfläche der Mikrosäulen (104; 204) mit einer Metallplattierung versehen ist.

8. Verfahren nach einem der Ansprüche 1 bis 7,
   **dadurch gekennzeichnet, dass** die Gruppe der Mikrosäulen (104; 204) Aussparungen (205) mit einem vorgegebenen Abstand besitzt.

9. Verfahren nach einem der Ansprüche 1 bis 8,
   **dadurch gekennzeichnet, dass** die Mikrosäulen (104; 204) so ausgebildet sind, dass sie einen äquivalenten Durchmesser (D) von 50 nm bis 1 $\mu$m und eine Höhe von 100 nm bis 10 $\mu$m besitzen.

10. Verfahren nach einem der Ansprüche 1 bis 9,

**dadurch gekennzeichnet, dass** das funktionelle Element (100) als Folie oder Platte ausgebildet ist.

11. Verfahren nach einem der Ansprüche 1 bis 10,
**dadurch gekennzeichnet, dass** die Mikrosäulen (104; 204) und die darunterliegende Matrix (102; 202) aus einem Material hergestellt sind, dessen Hauptbestandteil ausgewählt ist unter Polymethylmethacrylat (PMMA), Cycloolefin-Polymeren, Polystyrol, Polycarbonat, Polyethylenterephthalat (PET), Polymilchsäure, Polypropylen, Polyethylen, Polyvinylalkohol, ABS-Harz, AS-Harz, Polyamid, Polyacetal, Polybutylenterephthalat, glasverstärktem Polyethylenterephthalat, modifiziertem Polyphenylenether, Polyvinylchlorid, Polyphenylensulfid, Polyetheretherketon, flüssigkeitsähnlichen Polymeren, Fluorharzen, Polyacrylat, Polyethersulfon, Polyamidimid, Polyetherimid, thermoplastischem Polyimid, Phenolharzen, Melaminharzen, Harnstoffharzen, Epoxyharzen, ungesättigten Polyesterharzen, Siliconharzen, Diallylphthalat-Harzen, Polyamid-Bismaleinimid-Harzen und Polybiamid-Triazol-Harzen.

## Revendications

1. Procédé de fabrication d'un substrat fonctionnel (100) équipé d'un groupe de micro-piliers colonnaires (104; 204) en polymère organique agencé sur une matrice (102; 202) en polymère organique disposée sur un élément de support (401) selon un schéma déterminé au préalable, dans lequel les micro-piliers (104) ont un diamètre équivalent (D) compris entre 10 nm et 500 $\mu$m, une hauteur (H) comprise entre 50 nm et 5000 $\mu$m et un rapport d'aspect (H/D) égal à 4 ou plus,
et dans lequel le diamètre équivalent (D) des extrémités des micro-piliers (104; 204) est inférieur à celui de la surface inférieure, comprenant les étapes suivantes qui consistent à :

- appliquer un film mince (404) en résine thermoplastique ou en résine thermodurcissable non durcie sur un élément de support (401),
- presser un micro-moule (405) comprenant un groupe de piqûres selon le schéma déterminé au préalable contre le film mince (404), et
- séparer le micro-moule (405) du film mince (404), ce qui permet d'étirer la partie du film mince (404) pressée dans les piqûres pour former le groupe de micro-piliers (104; 204) ayant un diamètre équivalent légèrement inférieur au diamètre interne des piqûres, mais ayant une longueur supérieure à la profondeur des piqûres,
- ces étapes étant suivies d'un durcissement quand une résine thermodurcissable non durcie est utilisée.

2. Procédé selon la revendication 1,
**caractérisé en ce que** le rapport d'aspect (H/D) des micro-piliers (104 ; 204) est compris dans la plage de 4 à 100.

3. Procédé selon la revendication 1 ou 2,
**caractérisé en ce que** le rapport d'aspect (H/D) des micro-piliers (104 ; 204) est compris dans la plage de 8 à 30.

4. Procédé selon l'une quelconque des revendications 1 à 3, **caractérisé en ce que** les micro-piliers (104, 204) possèdent une partie évasée à partir de la base en contact avec la matrice (102 ; 202) vers l'extrémité.

5. Procédé selon l'une quelconque des revendications 1 à 4, **caractérisé en ce qu'**au moins une partie des micro-piliers (104 ; 204) possède à son extrémité une partie agrandie en direction du diamètre.

6. Procédé selon l'une quelconque des revendications 1 à 5, **caractérisé en ce qu'**au moins la surface des micro-piliers (104 ; 204) est constituée d'une substance composée essentiellement d'un polymère organique hydrophobe et/ou oléophobe.

7. Procédé selon l'une quelconque des revendications 1 à 6, **caractérisé en ce qu'**au moins une partie de la surface des micro-piliers (104 ; 204) est dotée d'un revêtement métallique.

8. Procédé selon l'une quelconque des revendications 1 à 7, **caractérisé en ce que** le groupe de micro-piliers (104 ; 204) comprend des espacements (205) ayant un écartement déterminé au préalable.

9. Procédé selon l'une quelconque des revendications 1 à 8, **caractérisé en ce que** les micro-piliers (104 ; 204) ont un diamètre équivalent (D) compris entre 50 nm et 1 $\mu$m et une hauteur comprise entre 100 nm et 10 $\mu$m.

10. Procédé selon l'une quelconque des revendications 1 à 9, **caractérisé en ce que** le substrat fonctionnel (100) se présente sous la forme d'un film ou d'une feuille.

11. Procédé selon l'une quelconque des revendications 1 à 10, **caractérisé en ce que** les micro-piliers (104 ; 204) et la matrice sous-jacente (102 ; 202) sont constitués d'un matériau, dont le principal composant est sélectionné parmi le polyméthacrylate de méthyle (PMMA), les polymères de cyclo-oléfines, le polystyrène, le polycarbonate, le téréphtalate de polyéthylène (PET), l'acide polylactique, le polypropylène, le polyéthylène, l'alcool polyvinylique, la résine ABS, la résine AS, le polyamide, le polyacétal, le téréphtalate de polybutylène, le téréphtalate de polyéthylène renforcé de fibres de verre, le polyphénylène éther modifié, le polychlorure de vinyle, le polysulfure de phénylène, le polyétheréthercétone, les polymères de type liquide, les fluororésines, le polyacrylate, le polyéther sulfone, le polyamideimide, le polyétherimide, le polyimide thermoplastique, les résines phénoliques, les résines mélamines, les résines uréiques, les résines époxydes, les résines en polyester non saturé, les résines silicones, les résines de phtalate de diallyle, les résines de polyamide bismaléimide et les résines de polybiamide trizole.

# FIG. 1

## FIG. 2

## FIG. 3

FIG. 4a — 401

FIG. 4b — 402

FIG. 4c — 403

FIG. 4d — 404

FIG. 4e — 409 / 405

FIG. 4f — 406

FIG. 4g — 407 / 408

FIG. 4h — 408 / 404

FIG. 4i — 408 / 404

100

## FIG. 5

## FIG. 6

# FIG. 7

# FIG. 8

# FIG. 9

# FIG. 10

## FIG. 11

609

607

606

## FIG. 12

VISIBLE
INCOMING LIGHT
(WAVELENGTH λ 1, λ 2,···)

λ 1 (BLUE)     λ 2 (YELLOW)

704

700

702

P1     P2

# FIG. 13

OUTGOING LIGHT ⬆

708 — 
707 — 
} 705

INCOMING LIGHT
706 — ( $\lambda$ ) ⬆

# FIG. 14

OUTGOING LIGHT ⬆ AIR
( $\lambda 1$, $\lambda 2$, $\lambda 3 \cdots$ )

708 — 
707 — 
706 — 

INCOMING LIGHT ⬆
( $\lambda 1$, $\lambda 2$, $\lambda 3 \cdots$ )

# FIG. 15

OUTGOING LIGHT ⬆ AIR
( $\lambda 1$, $\lambda 2$, $\lambda 3 \cdots$ )

708 — 
} 705
707 — 
706 — 

INCOMING LIGHT ⬆
( $\lambda 1$, $\lambda 2$, $\lambda 3 \cdots$ )

EP 1 416 303 B1

FIG. 16a

803
801
800

FIG. 16b

803
801
800

FIG. 16c

804
800

ELECTROLESS
NICKEL PLATING

FIG. 16d

805
800

27

*FIG. 17a*    401

*FIG. 17b*    404

*FIG. 17c*    405

*FIG. 17d*    406

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Applied Physics,* 2002, vol. 71 (10), 1251-1255
- *Journal Vacuum Science and Technology B,* 1999, vol. 17 (6), 3197-3202
- **Hase et al.** Smart Structures and Materials 2000: Smart Electronic and MEMS. *Proceedings of the SPIE,* 2000, vol. 3990, 314-325